# EUROPÄISCHE PATENTSCHRIFT

(11) **EP 1 834 616 B1**
(45) Veröffentlichungstag und Bekanntmachung des Hinweises auf die Patenterteilung: **24.04.2013**
(21) Anmeldenummer: 06005318.8
(22) Anmeldetag: 15.03.2006
(51) Int. Cl.: A61F 9/008, B23K 26/06, B23K 26/40

(54) **Steuerprogramm für die zeitliche und räumliche Steuerung von Laserpulsen**
Control program for the temporal and spatial control of laser pulses
Programme de commande temporelle et spatiale d'impulsions laser

(43) Veröffentlichungstag der Anmeldung: 19.09.2007
(73) Patentinhaber: WaveLight GmbH, 91058 Erlangen (DE)
(72) Erfinder: Mrochen, Michael, 8606 Nänikon (CH)
(74) Vertreter: von Hellfeld, Axel

(56) Entgegenhaltungen:
- WO-A1-93/08877
- DE-A1- 10 334 109
- US-A1- 2003 023 233
- US-A1- 2003 069 566
- US-A1- 2004 243 111
- US-A1- 2005 107 773

## Beschreibung

Die Erfindung betrifft ein Verfahren zum Erzeugen eines Steuerprogramms für die zeitliche und räumliche Steuerung von Laserpulsen, die entsprechend einer Zielform oder Zielfläche in oder auf einem zu bearbeitenden Material zu positionieren sind.

In der Materialbearbeitung werden gepulste Lasersysteme vielfältig eingesetzt, um Änderungen auf oder in dem Material zu erzielen. Nachfolgend soll beispielhaft für eine solche Materialbearbeitung eine Anwendung in der Ophthalmologie herausgegriffen werden, für die sich die vorliegende Erfindung besonders eignet.

Die Kornea des menschlichen Auges kann bekanntermaßen mit Laserpulsen im UV-Bereich neu geformt werden. Bekannt ist dabei insbesondere das LASIK-Verfahren.

Bei diesem Verfahren werden zum Beispiel Laserpulse zeitlich und räumlich von einem Rechner so gesteuert, dass eine Vielzahl von Pulsen nebeneinander auf oder in dem Material positioniert werden. Die Wirkungen der Laserpulse an den Orten ihrer Positionierung, wobei die Positionierung zum Beispiel durch Fokussierung erfolgen kann, sind unterschiedlicher Art. Bei der LASIK-Technologie kennt man zum Beispiel photodisruptive Wirkungen und ablative Wirkungen der Laserpulse. Photodisruptive Wirkungen der Laserpulse werden zum Beispiel beim Schneiden eines Flaps (Deckelchens) in der Kornea ausgenutzt. Dabei werden Laserpulse mit Pulslängen im Femtosekundenbereich (also kleiner als eine Pikosekunde) im Korneagewebe zeitlich und räumlich nacheinander so positioniert, dass die photodisruptive Wirkung letztlich ein Flap aus dem Korneagewebe herausschneidet. Ein mechanisches Mikrokeratom ist bei dieser Technik nicht erforderlich.

Eine photoablative Wirkung wird beim LASIK-Verfahren in einem zweiten Schritt eingesetzt. Bei diesem Schritt wird zunächst der wie vorstehend beschrieben erzeugte Flap über eine Gelenkstelle zur Seite geklappt und sodann werden die Spots des Laserstrahls rechnergesteuert Punkt für Punkt über das freiliegende Stroma der Kornea geführt, um Stromagewebe abzutragen und so nach Rückklappen des Flaps der Kornea eine neue Form zu geben, mit der zuvor vorhandene Abbildungsfehler und Aberrationen höherer Ordnung entfernt oder zumindest verringert werden.

In der Materialbearbeitung kennt man zum Beispiel auch photothermische Wirkungen von positionierten Laserpulsen, um zum Beispiel Materialumwandlungen zu erreichen.

Den vorstehend skizzierten Techniken ist gemeinsam, dass die Laserpulse mit einem Rechner zeitlich und räumlich in oder auf dem zu bearbeitenden Material gesteuert werden. Die räumliche Steuerung erfolgt zum Beispiel über einen beweglichen Spiegel nach dem Galvanometerprinzip. Dies ist als solches dem Fachmann bekannt.

Werden Laserpulse, insbesondere fokussiert, in der oben beschriebenen Weise in einem Material positioniert, zum Beispiel im Korneagewebe zur Erzeugung eines Flaps, dann werden die einzelnen Stellen, an denen die Laserpulse gesetzt werden, vom Wirkungsbereich der Laserstrahlung im Material abhängen. Hat zum Beispiel ein bestimmter Laser im Korneagewebe eine photodisruptive Wirkung in einem Bereich von wenigen Mikrometern, dann werden die Laserpulse typischerweise mit Abständen in der gleichen Größenordnung nebeneinandergesetzt, um durch den photodisruptiven Effekt letztlich einen relativ glatten Schnitt zu erzeugen. Zielsetzungen dabei sind insbesondere: 1. eine geeignete, ausreichende Überlappung der Wirkungsbereiche der einzelne fokussierten Laserpulse; 2. eine leichte Trennbarkeit des Materials nach der Schnittbildung; 3. eine glatte Schnittoberfläche; 4. keine thermischen Nebeneffekte; 5. eine schnelle Schnitterzeugung. Zur Erreichung all dieser, teilweise gegenläufigen Zielsetzungen erfolgt eine Optimierung der Positionen der Foci der Einzelpulse relativ zueinander. Dabei kann die Wirkung der Laserpulse richtungsabhängig sein, je nach Eigenschaften der Laserstrahlung und ihrer Fokussierung und Eigenschaften des Materials. Zum Beispiel kann die Ausdehnung der Wirkung eines Laserpulses in seiner Strahlungsrichtung größer sein als quer dazu.

Die US 2003/0023233 A1 beschreibt ein Verfahren für insbesondere die Ablation der Kornea des Auges, bei dem das abzutragende Volumen in horizontale Scheiben aufgeteilt und sodann geprüft wird, ob die Punkte eines regelmäßigen Gitters in diesen Scheiben liegen. Eine Anpassung der Gitterkonstanten an die anisotrope Wirkung des Lasers findet nicht statt.

Die US 2003/0069566 A1 beschreibt die Ablation von kornealem Gewebe unter Verwendung von Polynomen höherer Ordnung zur Erzeugung glatter Schnittflächen. Dokument DE 10334109 beschreibt die Berechnung eines Lentikel-Schnittsmittels Transformation der Schnittflächen in ein zweidimensionales Grid.

Die Erfindung hat das Ziel, bei der Erzeugung eines Steuerprogramms der eingangs genannten Art den rechnerischen Aufwand bei hoher Ablationsqualität zu reduzieren.

Die erfindungsgemäße Lösung dieser Aufgabe ist im Patentanspruch 1 beschrieben. Vorteilhafte Ausgestaltungen sind in den abhängigen Ansprüchen beschrieben.

Die Erfindung stellt also einen mathematischen Ansatz bereit zur Erzeugung eines Steuerprogramms für einen konkreten physikalischen Zweck, nämlich die Erzeugung von Zielformen oder Flächen in einem Material. In Bezug auf die LASIK-Technik bedeutet dies zum Beispiel, dass mit dem Steuerprogramm Femtosekunden-Laserpulse im Korneagewebe fokussiert werden und Puls für Puls der Ort dieser Fokussierung so geändert wird, dass photodisruptiv ein Flap (als Zielform) geschnitten wird. Analog gilt dies für anderen Anwendungen von Laserstrahlung, zum Beispiel der oben erläuterten Ablation oder auch thermischen Umwandlungen auf oder in Materialien, zum Beispiel bei nicht-medizinischen Anwendungen.

Ist der von jedem Laserimpuls erzielte Wirkungsbereich auf oder in dem Material richtungsabhängig, dann lehrt die Erfindung, gemäß der das Punktgitter in Richtung der Laserpulse eine andere Gitterkonstante hat als in einer Richtung senkrecht dazu, eine besonders elegante (einfache) Erzeugung des Steuerprogramms.

Ein besonders geeignetes Gitter für die genannten Zwecke ist das hexagonale Gitter.

Nachfolgend werden Ausführungsbeispiele der Erfindung anhand der Zeichnungen näher erläutert. Es zeigt:
- Figur 1: schematische eine hexagonale Gitterstruktur mit Gitterkonstanten;
- Figur 2: eine Fläche einer hexagonalen Raumstruktur zur Definition von Punkten; und
- Figuren 3 und 4: Ausführungsbeispiele für die Bestimmung von Schusspositionen von Laserpulsen in Bezug auf ein hexagonales Gitter.

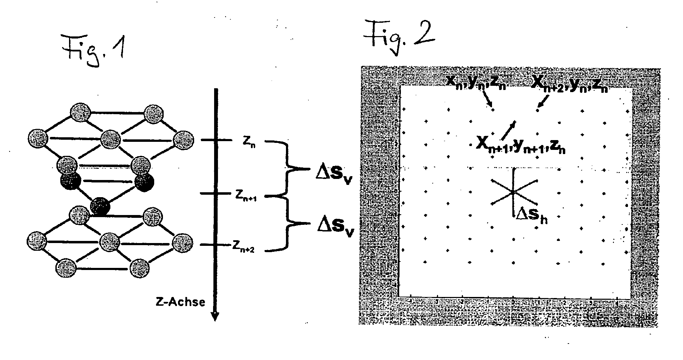

Figur 1 zeigt schematisch ein hexagonales Gitter. Im Folgenden werden cartesische Raumkoordinaten x, y, z verwendet, wobei die Laserstrahlrichtung parallel zur z-Achse ist. Die z-Achse steht senkrecht auf der x-y-Ebene. Soll Hornhautgewebe mit der Laserstrahlung bearbeitet werden, dann wird, wie allgemein üblich, die Horizontalebene der Hornhaut, auf welcher die optische Achse der Hornhaut etwa senkrecht steht, als x-y-Ebene bezeichnet. Die Laserstrahlung trifft dann etwa senkrecht zur x-y-Ebene auf die Hornhaut.

Die Figuren 1 und 2 zeigen die hier verwendeten Bezeichnungen. Figur 1 zeigt drei Ebenen in Richtung der z-Achse, bezeichnet mit Zₙ, Zₙ₊₁ und Zₙ₊₂. Der Abstand der Ebenen ist ΔSᵥ (v steht für vertikal). Das hexagonale Gitter, von dem in Figur 1 eine Zelle dargestellt ist, hat in der oberen Ebene der Zelle 6 Punkte. Entsprechendes gilt für die untere Ebene. In der Mitte dazwischen liegen drei Gitterpunkte in einer mittleren Ebene. Der Abstand zwischen der mittleren Ebene und einer der unteren bzw. oberen Ebenen ist hier mit "Gitterkonstante" bezeichnet. Der Abstand ist ΔSᵥ.

Figur 2 zeigt eine Draufsicht (in Richtung der z-Achse gemäß Figur 1) auf die Gitterstruktur und die Bezeichnung der Punkte in der Gitterebene Zₙ. Die Gitterkonstante in horizontaler Richtung ist der Abstand benachbarter Punkte und mit ΔSₕ bezeichnet.

Der Wirkungsbereich der Laserstrahlung kann in den verschiedenen Raumrichtungen x, y, z unterschiedlich sein. Entsprechend können unterschiedliche Gitterpunktabstände in den Richtungen in Abhängigkeit von den Wirkungsradien der Laserstrahlung in diesen Richtungen gewählt werden. Typischerweise wird der Wirkungsradius in z-Richtung unterschiedlich sein von den Wirkungsradien in der x-y-Ebene. Entsprechend können die Gitterabstände ΔSᵥ und ΔSₕ gewählt werden.

Bei Erzeugung des Steuerprogramms für die zeitliche und räumliche Steuerung von sukzessive auf oder im Material platzierten Laserpulsen wird die hexagonale Raumstruktur gemäß den Figuren 1 und 2 zugrundegelegt, d.h. die räumlichen Gitterpunkte sind die Stellen, an denen zum Beispiel der Fokus der Laserpulse positioniert wird.

Soll bei Anwendung in der refraktiven Augenchirurgie, zum Beispiel zur Erzeugung eines Flaps, die Form des Flaps bestimmt werden und entsprechend dann das Steuerprogramm für die Laserpulse erzeugt werden, lässt sich dies mathematisch mit zylinder-, kugel-, oder karthesischen Koordinaten durchführen.

Bei einer klinischen oder technischen Anwendung wird dann der Raum diskretisiert entsprechend den Gitterpunkten, zum Beispiel gemäß dem hexagonalen Raumgitter. Die Gitterpunkte entsprechen den Laserschusspositionen. Die zeitliche Steuerung durch den Rechner entspricht der Repititionsrate des gepulsten Lasers.

Als Zielform bzw. Zielfläche wird hier die im Material, zum Beispiel der Kornea, zu schneidende Form verstanden, zum Beispiel ein Flap (Deckel) bei Anwendungen in der Lasik-Technik. Unter Zugrundelegung des Gitters werden nun diejenigen Laserschusspositionen (Gitterpunkte) ermittelt, welche die vorgegebene Zielform (auch als Soll-Form zu bezeichnen) am besten wiedergeben.

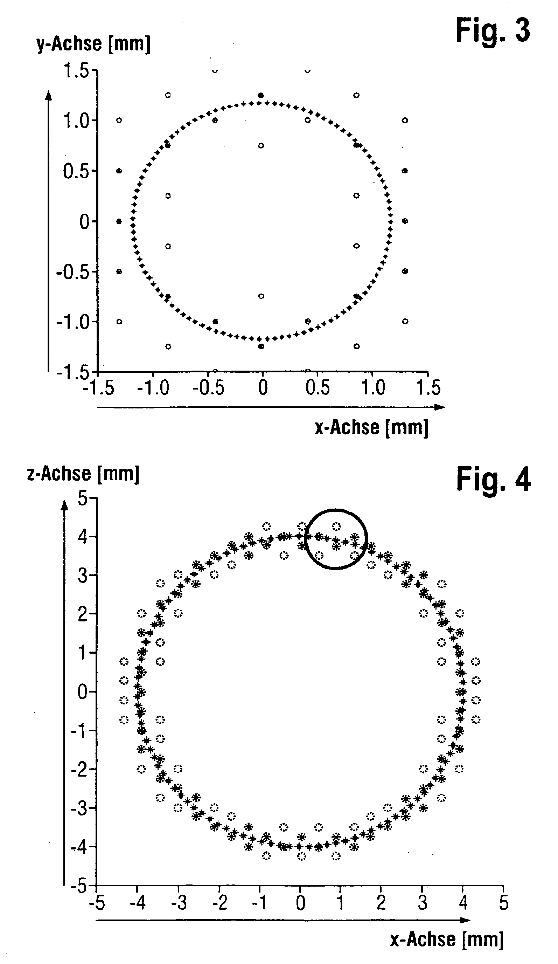

Dies ist für die Ebene in Figur 3 dargestellt. Figur 3 zeigt die x-y-Ebene des Gitters, wobei die Gitterpunkte als Kreise ohne Füllung dargestellt sind. Die gefüllten Kreise sind diejenigen ausgewählten Gitterpunkte, welche die Zielform (hier ein Kreis) am besten beschreiben. Zur Erläuterung des Prinzips ist die Gitterkonstante in Figur 3 sehr groß gewählt und entsprechend ist die Anpassung der ausgewählten Gitterpunkte an die Zielform relativ grob. Schon eine Verringerung der Gitterkonstanten auf die Hälfe würde eine sehr viel bessere Übereinstimmung zwischen der Zielform und den ausgewählten Gitterpunkten ergeben. Die Darstellung gemäß Figur 3 ist auf die Fläche beschränkt. Die Erweiterung auf den 3-dimensionalen Raum erfolgt analog.

Figur 4 zeigt ein Ausführungsbeispiel mit erhöhter Auflösung durch Verringerung der Gitterkonstanten. Die Zielform ist kreisförmig. Die Gitterpunkte sind wieder mit leeren Kreisen dargestellt, wobei diejenigen Gitterpunkte, welche für die Anpassung an die Zielform ausgewählt sind, jeweils mit einem Sternchen gefüllt sind. Die Übereinstimmung mit der Zielform ist deutlich.

Die Vorgabe des Gitters und insbesondere der Gitterkonstanten sowie die Auswahl der geeigneten Punkte des Raumgitters hängt von der Zielform und der gewünschten Schnittqualität ab. Die Schnittqualität entspricht der Genauigkeit, mit der die ausgewählten Gitterpunkte die Zielform wiedergeben. Als Maß für die Schnittqualität lässt sich zum Beispiel die mittlere quadratische Abweichung der Raumgitterpunkte von der mathematisch definierten Zielform (bzw. Zielfläche) heranziehen.

Ein weiteres Bewertungskriterium für die Schnittqualität kann zum Beispiel den Wirkungsradius der einzelnen Laserschüsse heranziehen. Je kleiner der Wirkungsradius, gegebenenfalls richtungsabhängig, ist, umso feiner muss in der Regel das Raumgitter gewählt werden, also umso kleiner muss die Gitterkonstante sein. Entsprechend steigt der Rechenaufwand. Die Schnittqualität ergibt letztlich eine bestimmte Rauhigkeit oder Welligkeit der erzeugten Oberflächen.

Bei Fokussierung von Laserstrahlung im Material, zum Beispiel zur Ausnutzung photodisruptiver Effekte, kommt es in bestimmten Materialien, wie zum Beispiel der Hornhaut, zu Bläschenbildung (Kavitationen). Solche Kavitationsblasen haben eine Lebensdauer, die in Regel deutlich länger ist als der zeitliche Abstand zwischen zwei aufeinanderfolgenden Laserpulsen. Fokussiert man deshalb einen nachfolgenden Laserpuls in einer solchen Kavitationsblase, dann kann es zu höchst unerwünschten Effekten kommen. Deshalb werden zeitlich aufeinanderfolgende Laserschüsse nicht in benachbarten Gitterpunkten platziert, sondern in weiter entfernten Gitterpunkten derart, dass keine unerwünschten Störungen durch Kavitationsblasen auftreten. Insgesamt werden aber alle ausgewählten Punkte des Raumgitters, welche die Zielform wiedergeben, abgearbeitet, d.h. in jedem ausgewählten Raumpunkt wird ein Laserpuls platziert. Alternativ zur vorstehend beschriebenen entfernten Streuung der Gitterpunkte ist es auch möglich, die Gitterkonstante zu variieren, um Störungen durch Kavitationsblasen zu vermeiden.

Der Algorithmus zur Erzeugung des Steuerprogramms für die zeitliche und räumliche Steuerung der Laserpulse hat dann folgenden allgemeinen Ablauf:

Zunächst wird die Zielform, welche mit den Laserpulsen geschnitten werden soll, mathematisch definiert. Dies kann durch einen analytischen Ausdruck oder numerisch geschehen. Sodann wird das Raumgitter, zum Beispiel das hexagonale Raumgitter, vorgegeben mit bestimmten Gitterkonstanten in Richtung der z-Achse (Δsᵥ) und in der x-y-Ebene (Asₕ). Diese Gitterkonstanten werden ausgewählt in Abhängigkeit von dem Wirkungsradius der Laserpulse, der Geometrie der Zielform, und der gewünschten Schnittqualität.

Sodann werden diejenigen Raumgitterpunkte ermittelt, welche die Zielform am besten wiedergeben (die Zielform kann auch 2-dimensional sein).

Sodann erfolgt eine Bewertung der Schnittqualität und ob sie vorgegebenen Kriterien hinsichtlich der Schnittqualität und Schnittzeit genügt, d.h. die Übereinstimmung zwischen den ausgewählten Raumpunkten und der Zielform wird analysiert.

Sodann wird der zeitliche Ablauf der sukzessiven Laserpulse in Abhängigkeit von dem Ort definiert, zum Beispiel um die oben erläuterten Probleme durch Kavitationen zu vermeiden.

Entsprechend wird dann das Steuerprogramm generiert. Das Steuerprogramm wird dann an ein Lasersystem mit x-y-z-Abtasteinheit übergeben und entsprechend erfolgt die Abarbeitung des Steuerprogramms mit zeitlich und räumlich gesteuerten Laserpulsen zur Erzeugung der Zielform im Material.

## Patentansprüche

1. Verfahren zum Erzeugen eines Steuerprogramms für die zeitliche und räumliche Steuerung von Laserpulsen, die entsprechend einer Zielform oder Zielfläche in oder auf einem zu bearbeitenden Material zu fokussieren und zu positionieren sind,
mit folgenden Schritten:
- Vorgabe eines Punktgitters mit vorgegebenen Gitterkonstanten,
- Auswahl derjenigen Gitterpunkte, die eine Übereinstimmung mit der Zielform oder Zielfläche gemäß einem vorgegebenen Übereinstimmungskriterium ergeben,
- Festlegen, dass die Laserpulse gemäß den ausgewählten Gitterpunkten zu positionieren sind,
wobei die Gitterkonstanten in Abhängigkeit von einem richtungsabhängigen photodisruptiven Wirkungsradius der Laserpulse in dem Material gewählt werden,
**dadurch gekennzeichnet, dass** das Punktgitter in Richtung der Laserpulse eine andere Gitterkonstante hat als in einer Richtung senkrecht dazu.

2. Verfahren nach Anspruch 1, **dadurch gekennzeichnet, dass** das Punktgitter hexagonal ist.

## Claims

1. A method for the generation of a control program for the time or space control of laser pulses which are to be focused and positioned according to a target shape or target area in or on a material to be processed,
with the following steps:
- specifying a point lattice with pregiven lattice constants,
- selecting those lattice points which result in a match with the target shape or the target area according to a pregiven coincidence criterium,
- establishing that the laser pulses are to be positioned according the selected lattice points,
wherein the lattice constants are selected depending on a direction-dependent photodisruptive action radius of the laser pulses in the material, **characterised in that** the point lattice has a lattice constant in the direction of the laser pulses which is different from that in a direction perpendicular to it.

2. The method according to Claim 1, **characterised in that** the point lattice is hexagonal.

## Revendications

1. Procédé pour produire un programme de commande destiné à la commande temporelle et spatiale d'impulsions laser qui doivent être focalisées et positionnées conformément à une forme de cible ou à une surface de cible dans ou sur un matériau à traiter,
le procédé comprenant les étapes suivantes :
- définir préalablement un réseau de points présentant des constantes de réseau prédéfinies,
- sélectionner les points de réseau qui conduisent à une concordance avec la forme de cible ou la surface de cible conformément à un critère de concordance prédéfini,
- spécifier que les impulsions laser sont à positionner conformément aux points de réseau sélectionnés,
les constantes de réseau étant sélectionnées en fonction d'un rayon d'action photodisruptif directionnel des impulsions laser dans le matériau,
**caractérisé en ce que** le réseau de points présente une constante de réseau dans la direction des impulsions laser, qui est autre que dans une direction perpendiculaire à celle-ci.

2. Procédé selon la revendication 1, **caractérisé en ce que** le réseau de points est hexagonal.
